# EUROPEAN PATENT SPECIFICATION

(11) **EP 0 317 804 B1**
(45) Date of publication and mention of the grant of the patent: **04.10.1995**
(21) Application number: 88118299.2
(22) Date of filing: 03.11.1988
(51) Int. Cl.: C07K 14/00, A61K 38/00, G01N 33/68, A61K 39/21, G01N 33/569, C12P 21/08

(54) **HIV peptides and methods for detection of HIV**
HIV-Peptide und Methoden für den Nachweis von HIV
Peptides de VIH et méthodes de détection de VIH

(30) Priority: 24.11.1987 US 124801
(43) Date of publication of application: 31.05.1989
(73) Proprietor: ABBOTT LABORATORIES, Abbott Park, Illinois 60064-3500 (US)
(72) Inventor: Sarin, Virender K., Libertyville IL 60048 (US); Knigge, Kevin M., Gurnee, IL 60031 (US)
(74) Representative: Modiano, Guido, Dr.-Ing.

(56) References cited:
- EP-A- 0 212 532
- EP-A- 0 231 914
- WO-A-86/02383

## Description

### BACKGROUND OF THE INVENTION

A retrovirus termed human immunodeficiency virus (HIV) is now known to be the etiologic agent in acquired immune deficiency syndrome (AIDS). Various isolates of this virus have been termed lymphadenopathy-associated virus (LAV), human T-cell lymphotropic virus type-III (HTLV-III) or AIDS-associated retrovirus (ARV). Although the modes of transmission for HIV are not completely understood, the most common forms of transmission of the HIV virus are through sexual contact, use of contaminated intravenous equipment and transfusions with contaminated blood products. Testing for HIV has become extremely important in diagnosing exposure to the virus and particularly for protecting blood products from contamination.

To detect exposure to HIV, levels of HIV antibodies (anti-HIV) are measured in serum, plasma, saliva or other biological samples of HIV patients or individuals at risk for AIDS. In the first generation of tests for HIV antibodies, inactivated crude or purified viral protein from lysates of HIV-infected cells are used to coat a solid phase. The lysate-coated solid phase is incubated with a biological sample suspected of containing anti-HIV, washed and then anti-human antibody tagged with a detectable label is added to the solid phase. The label, which may be an enzyme, radioisotope or fluorescent molecule is measured to determine presence of HIV antibodies in the sample.

The problem with using viral lysates as a source of HIV antigens is that the infectious nature of the HIV virus makes manufacturing the virus potentially hazardous. Also, HIV viral lysates may contain impurities which interfere with testing for HIV antibodies. Therefore, alternate sources of HIV antigens are needed which are safe and noninfectious. There is also a need for HIV antigens which are well-defined and do not cross-react with other non-HIV antibodies and other interfering materials contained in the sample to be tested.

Studies have shown that HIV antibodies to gp160 and gp120 are found in human saliva of asymptomatic, AIDS-related complex and AIDS patients. Archibald, et al., Blood, 67:831-834 (1986). These salivary antibodies represent mostly IgA immune response.

Various isolates of HIV have been cloned and their genetic structures established. Nucleotide and deduced amino acid sequences of the various regions of the HIV genome are published. However, the published sequences do not provide any information as to which portions of the HIV molecule, when synthesized as peptides, would have antigenic or immunogenic properties similar to the corresponding region of naturally-occurring HIV proteins.

The envelope (env) region of the HIV gene encodes an approximately 856 residue precursor protein with various potential glycosylation sites. The precursor glycoprotein corresponds to a molecular weight of 160,000 daltons (gp160) and is processed at the -Lys Arg- pair to yield an N-terminal protein of 480 amino acids (gp120) and a 345 amino acid protein (gp41).

It has been demonstrated by radioimmune precipitation followed by polyacrylamide gel electrophoresis (RIP-PAGE) as well as by enzyme-linked immunosorbent assay (ELISA) that sera from AIDS and AIDS-related complex (ARC) patients react with env and gag gene encoded proteins of HIV. These proteins include but are not limited to gp160 gp120, gp41, p55, p36, p24, p18 and p12.

In European Patent Application No. 227,169, Berman et al. disclose several polypeptides mimicking amino acid sequences of AIDS-related viral proteins. An immunochemical reagent is made by combination of two or more of the synthetic polypeptide sequences selected from the following group: a polypeptide sequence mimicking at least one antigenic determinant of the gag antigen of HIV, a polypeptide sequence mimicking at least one antigenic determinant of the glycoprotein gp120 of HIV and a polypeptide sequence mimicking at least one antigenic determinant of the glycoprotein gp41 of HIV. Berman et al. state that the polypeptide sequences disclosed should be substantially free of the naturally occurring gag, gp120 and gp41 proteins of HIV even though these reagents may optionally contain naturally occurring HIV proteins. The Berman et al. disclosure provides no indication as to which HIV antigen or epitope would be important for detecting salivary antibodies.

In Wong-Staal, et al., U.S. Pat. Applic. Ser. No. 779,431, a 15 amino acid long peptide from gp120 is described. This peptide is useful as an immunogen in the production of monoclonal antibodies and in an ELISA assay.

In Proc. Nat'l Acad. Sci. USA, 84:2479-2483 (1987), Palker, et al. disclose a synthetic peptide derived from the COOH-terminal region of gp120 containing 15 amino acids. This relatively short peptide was used to evaluate reactivity of HIV-positive patients' antibodies and as an immunoadsorbent to evaluate functional importance of human antibody response to the COOH terminus of gp120.

In Cosand, U.S. Patent No. 4,629,783, peptides having sequences mimicking short regions of gp120, gp41 and p24 are disclosed. Those peptides, which can be used for detection of anti-HIV in blood screening procedures, mimic proteins encoded by the gag or env regions of the viral genome. Preferably, the peptides disclosed by Cosand contain fewer than 25 amino acids.

In Montagnier et al., WO 86/02383, a LAV envelope antigen having a molecular weight of about 110,000 or antigens of lower molecular weight (less than 200 amino acids) derived from the preceding one are disclosed as useful in the diagnosis of LAV antibodies in the sera of patients.

In Gallo et al., EP-A-0 231 914, synthetic peptides containing portions of the epitopic sequence HTLV env (460-550) are disclosed which peptides are useful as reagents in immunoassays for detection of AIDS antibodies, as immunogens for eliciting polyclonal or monoclonal antibodies against AIDS virus env protein and as components in AIDS vaccines.

In Chan et al., EP-A-0 212 532, a method for producing fusion proteins is disclosed wherein the fusion protein may contain an AIDS virus env protein. Particular immunoreactive AIDS virus env protein fragments are disclosed.

### SUMMARY OF THE INVENTION

We have developed novel gp160 peptides which can be used for detecting HIV antibodies in biological samples, for producing HIV antibodies and for vaccines. These peptides substantially mimic regions of gp160 or its gp120 fragment and provide noninfectious and pure sources of HIV antigens.

First, a short peptide (Peptide I) corresponding to amino acids 490-517 of gp120 encoded in the region between base pairs (bp) 7221-7305 [Sanchez-Pescador, et al., Science, 227:484-492 (1985)] was synthesized for testing immunogenicity/antigenicity. This peptide sequence is shown below: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X,
wherein Y corresponds to -H or an amino acid, and X may be -OH, -NH₂ or -NR₁R₂ (wherein R corresponds to an alkyl group).

The amino acid sequence of Peptide II corresponds to amino acids 482-517 of gp120 encoded in the region between bp 7197-7305 [Sanchez-Pescador, et al., supra]. The amino acid sequence for Peptide II is shown below: 
(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X,
wherein Y and X are the same as described above for Peptide I.

Peptide III extends from amino acids 455 to 511 of gp120 encoded in the region between bp 7165-7335 [Muesing, et al., Nature, 313:450-458 (1985)]. The amino acid sequence of this peptide is shown below: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X,
wherein Y and X are as explained for Peptide I.

These peptides can be used as sources of HIV antigens alone or in combination or may be linked to larger carrier molecules. It is preferred that one or more of the Peptides I-III be used in combination with other antigens and epitopes, particularly other HIV antigens such as p24 and gp41.

The peptides of the invention can be synthesized by a number of methods including synthesis in solution or by solid phase peptide methodology using stepwise or fragment coupling protocols. They can also be synthesized enzymatically or made as fused proteins or peptides by recombinant DNA methodology. Regions of genes coding for these peptides may also be synthesized, cloned and expressed using recombinant DNA technology, and the sequences may be subcloned and expressed in suitable expression systems such as E.coli, yeast or mammalian cells. The peptides described herein may be combined with other epitopes or antigens of HIV, with or without carrier molecules, and expressed by recombinant DNA methods or made by synthetic methods as fusion proteins. Substitution, deletion or addition analogs of the peptides of the invention can also be made by methods well known to those skilled in the art.

The peptides corresponding to the various examples herein can be used alone, in combination with one another or in combination with other antigens of interest. For example, various of these peptides can be used as physical mixtures or chemically coupled to each other with or without spacer molecules. It is also possible to couple peptides chemically or physically with carrier peptides, proteins or supports. These peptides can be used with other HIV antigens or epitopes such as gp41, gp120, p55, p24 and others. The peptides, particularly Peptide III, can be coupled to carrier molecules like thyroglobulin or BSA and used as antigens alone or in combination with other epitopes or antigens.

### DETAILED DESCRIPTION OF THE INVENTION

The following examples illustrate methods of making the peptides, as well as methods of using the peptides as sources for pure, well-defined, noninfectious HIV antigens.

### Example 1

This example illustrates the synthesis of Peptide I on a resin support by stepwise solid phase synthesis starting with the carboxy-terminal residue. A procedure such as the procedure described in Barany and Merrifield, The Peptides, 2:1984, Gross, E., and Meinehofer, J., Eds., Academic Press, New York, N.Y. (1980) can be used for this synthesis. A Boc-L-Arg(Tos)-OCH₂-Pam resin was transferred to a reaction vessel of an Applied Biosystems Synthesizer, Model 430A, available from Applied Biosystems, Foster City, California. Protected amino acids were coupled in a stepwise manner to the resin support by preformed symmetric anhydride chemistry, except in the cases of arginine, asparagine and glutamine addition where the DCC/HOBT protocol described by Konig and Geiger, Chem. Ber., 103:788-798 (1970) was employed. All amino-terminal residues were protected by t-butyloxy carbonyl (t/BOC-linkage) and side chains of various amino acid residues were protected by the following groups: Arg, Tos; Lys, 2-ClZ; Glu, OBZl; Thr, Bzl; Tyr, 2-BrZ.

The fully protected peptide-resin (0.7 g) was allowed to swell in methylene chloride (CH₂Cl₂) for 5 minutes. The N^{α}-Boc protecting groups were removed using 60% trifluoroacetic acid (TFA/CH₂Cl₂) deprotection, CH₂Cl₂ washes, 10% N,N diisopropylethylamine (DIEA/CH₂Cl₂) neutralization and finally washing with CH₂Cl₂ again. The resin was dried in vacuo. The peptide resin so obtained was treated with 9 ml of anhydrous hydrofluoric acid (HF) to which 1 ml of p-cresol had been added, for 60 minutes at 0°C. The HF was distilled off in vacuo at 0°C. The cleaved free peptide and resin were washed 3 times with 15 ml aliquots of diethyl ether, and the peptide was extracted by means of 3 extractions with 15 ml of 40% aqueous acetic acid. The aqueous extracts were combined and washed three times with 10 ml aliquots of diethyl ether, whereupon the aqueous layer was lyophilized to provide the crude peptide for purification. The polypeptide was purified by reversed-phase high performance liquid chromotography (HPLC) on C₄ columns employing gradients of 0.1% TFA/water (A) and 100% acetonitrile (B) as the solvent systems at a 1 ml/min. flow rate for the analytical (Vydac-214-TP54, Vydac Separation Group, Hesperia, California) or 3 ml/mm flow rate for the semi-preparative (Vydac-214-TP510) columns. The gradient used was: 
1 min                    20 min                    1 min
20%B    →    20%B    →    25%B    →    20%B
The polypeptide elution from the HPLC column was monitored at 222 nm and 280 nm. The composition of the polypeptide was confirmed by hydrolysis in 6 N hydrochloric acid (HCl)/0.3% phenol at 150° C for 2 hours in vacuo and subsequently analyzed on a Beckman 6300 amino acid analyzer with a SICA 7000 A integration available from Beckman Instruments, LaBrea, California.

Peptides II and III were synthesized in a manner similar to the one described above. Methionine was used in the sulfoxide form, trytophan was protected by the formyl group (CHO); Ser, Bzl; Asp, OBZL. For peptide-resins containing methionine sulfoxide and formyl-tryptophan, peptides were deprotected and cleaved off the resin using "low high" HF protocols as described by Tam, et al., J. Am. Chem. Soc., 105:6442-6455 (1983). Desired peptides can also be synthesized using unprotected methionine and tryptophan with the appropriate uses of scavengers during deprotection and cleavage. All peptides were purified using reversed phase C₄ HPLC wih 0.1% aqueous TFA and a 100% acetonitrile gradient system.

Peptides I, II and III may be conjugated to larger carrier molecules such as bovine serum albumin (BSA), keyhole limpet hemocyanin (KLH) or thyroglobulin using water soluble carbodiimide or maleimido-benzoyl-N-hydroxysuccinimide (MBS) as described in Liu, et al., Biochem., 18:690-697 (1979), and Kitagawa, et al., J. Biochem., 92:585-590 (1982). These conjugates can then be used to raise sequence specific antibodies.

### Example 2

This example demonstrates a method of detecting HIV antibodies in biological samples using the peptides described in Example 1. Biological samples which can be tested by the methods described herein include blood preparations such as serum or plasma, urine and saliva. The peptides of the invention are particularly useful in a saliva test for the presence of IgA, IgG or IgM antibodies to HIV, especially when the peptides are combined with other HIV antigens.

Peptides I and III were tested side-by-side with recombinant DNA-derived HIV p24 and HIV gp41 to determine their usefulness as sources of HIV antigens in a method for detecting HIV antibodies in biological samples.

Four antigen solutions were prepared in 50 mM sodium carbonate, pH 9.5 as follows: Peptide I, 10 µg/ml; Peptide III, 6.25 µg/ml; recombinant DNA-derived HIV p24 protein, 1.64 µg/ml; and recombinant DNA-derived HIV gp41 protein, 125 units/ml. The p24 and gp41 proteins were made by methods described in copending U.S. Patent Application Serial No. 020,287, filed Feb. 27, 1987, assigned to the same assignee as that of the present invention.

One hundred microliters (100 µl) of each antigen solution were added to wells of a polystyrene microtiter plate available from Dynatech Laboratories, Alexandria, Virginia. Other solid phases which may be utilized in the methods described herein include beads, paper strips, microparticles, nitrocellulose membranes, polystyrene tubes or other suitable plastic or paper supports. The solution and the plate were incubated for 1 hour at room temperature after which the solution was removed and the plate washed five times with distilled water. Two hundred and fifty microliters of an overcoat solution consisting of 10% bovine serum albumin, 3% sucrose and 0.05% Tween® 20 in phosphate buffered saline (PBS) (0.01 M KH₂P0₄; 0.15 M NaCl: pH 7.2) were added to the wells. Following a 30 minute incubation, the overcoat solution was removed and the plate washed five times with distilled water. Coated plates were stored at 2-8°C for subsequent use.

The coated plates were used in an assay for detecting anti-HIV as follows: one hundred microliters of a serum sample diluted 1:800 or a saliva sample diluted 1:10 in a diluent consisting of 10% bovine serum albumin and 2% Tween® 20 in phosphate buffered saline (0.01 M KH₂P0₄; 0.15 M NaCl: pH 7.2) were added to wells of the coated microtiter plates. After a 1 hour incubation at room temperature, the sample was removed and the wells washed five times with distilled water. One hundred microliters of an antibody-enzyme conjugate (alkaline phosphatase:goat anti-human IgG or alkaline phosphatase:goat anti-human IgA) were added to the wells. The antibody-enzyme conjugate can also be an IgM antibody conjugate if IgM antibody is being detected.The antibody-enzyme conjugates are made as described in Engvall, et al., Biochim. Biophys. Acta, 251:427-434 (1971); Korn et al., J. Mol. Biol., 65:525-529 (1972); and Avrameas and Ternynck, Immunochemistry, 6:53-66 (1969). Following another 1 hour incubation at room temperature, the conjugate was removed and the wells washed five times with distilled water. One hundred microliters of a p-nitrophenylphosphate substrate solution were added to the wells and incubated at room temperature for 30 minutes. One hundred microliters of 2N NaOH were added to the wells to stop the reaction. Absorbance values of the wells were read at 405 nm. A positive result was determined at a cutoff value of 0.200 O.D. or greater, which was established based on the results generated from 100 negative and 50 positive paired serum and saliva samples. The results are set forth in Tables 1, 2 and 3.

The results indicate that all confirmed seropositive samples tested contained serum IgG to gp41, Peptide I and Peptide III, but the serum reactivity to p24 varied. In saliva, the IgG reactivity in seropositive samples varied for all antigens tested. Although the salivary IgA reactivity varied for p24 and gp41, all seropositive samples contained salivary IgA to Peptide I and Peptide III. For the confirmed negative samples, no reactivity was detected in either serum or saliva for any of the antigens tested. In general, the reactivity of Peptide III was greater than that of Peptide I.

These results demonstrate that Peptide I and Peptide III are specifically reactive with antibody to HIV. In addition, the results indicate that at least a portion of gp120 must be present when testing for antibodies to HIV in saliva samples.

**Table 1**

| Patient Diagnosis | Confirmed Seropos. | Microtiter Serum IgG | | | |
|---|---|---|---|---|---|
| | | p24 | gp41 | Peptide I | Peptide III |
| AIDS | Yes | 0.089 | >3.0 | 1.734 | >3.0 |
| AIDS | Yes | 0.034 | 2.009 | 0.981 | 1.730 |
| AIDS | Yes | 0.003 | >3.0 | 1.836 | 2.009 |
| AIDS | Yes | 0.006 | 2.105 | 0.301 | 0.873 |
| AIDS | Yes | 0.049 | 2.980 | 2.437 | >3.0 |
| AIDS | Yes | 0.097 | >3.0 | >3.0 | >3.0 |
| AIDS | Yes | 0.102 | 2.874 | 1.920 | 2.941 |
| AIDS | Yes | 0.031 | 2.541 | 1.337 | 2.226 |
| ARC | Yes | 0.198 | >3.0 | 2.011 | >3.0 |
| ARC | Yes | 0.157 | >3.0 | 1.990 | 2.798 |
| Asymptomatic | Yes | 2.983 | >3.0 | 1.933 | 2.322 |
| Asymptomatic | Yes | >3.0 | >3.0 | >3.0 | >3.0 |
| Asymptomatic | Yes | 0.895 | >3.0 | >3.0 | >3.0 |
| Asymptomatic | Yes | >3.0 | >3.0 | >3.0 | >3.0 |
| Asymptomatic | Yes | 1.971 | >3.0 | 2.909 | >3.0 |
| Hemophiliac | Yes | 0.301 | 2.179 | ND | 1.899 |
| Hemophiliac | Yes | 1.127 | 2.357 | ND | 0.207 |
| Hemophiliac | Yes | 1.193 | 1.715 | ND | 1.119 |
| Hemophiliac | Yes | 0.702 | 2.770 | ND | 2.248 |
| Hemophiliac | Yes | >3.0 | >3.0 | ND | >3.0 |
| Hemophiliac | Yes | 0.077 | 2.552 | ND | 0.558 |
| Hemophiliac | No | 0.051 | 0.086 | ND | 0.003 |
| Hemophiliac | No | 0.006 | 0.029 | ND | 0.079 |
| Hemophiliac | No | 0.018 | 0.068 | ND | 0.044 |
| High Risk | No | 0.005 | 0.076 | ND | 0.101 |
| High Risk | No | 0.018 | 0.006 | ND | 0.022 |
| Healthy Hetero. | No | 0.000 | 0.114 | 0.043 | 0.097 |
| Healthy Hetero. | No | 0.003 | 0.093 | 0.055 | 0.081 |
| Healthy Hetero. | No | 0.019 | 0.008 | 0.092 | 0.005 |
| Healthy Hetero. | No | 0.058 | 0.055 | 0.003 | 0.037 |

**Table 2**

| Patient Diagnosis | Confirmed Seropos. | Microtiter Saliva IgG | | | |
|---|---|---|---|---|---|
| | | p24 | gp41 | Peptide I | Peptide III |
| AIDS | Yes | 0.044 | 0.971 | 0.204 | 0.568 |
| AIDS | Yes | 0.013 | 0.111 | 0.123 | 0.228 |
| AIDS | Yes | 0.033 | 0.758 | 0.229 | 0.318 |
| AIDS | Yes | 0.022 | 0.014 | 0.041 | 0.059 |
| AIDS | Yes | 0.005 | 0.296 | 0.476 | 0.935 |
| AIDS | Yes | 0.041 | >3.0 | 2.037 | >3.0 |
| AIDS | Yes | 0.027 | 0.246 | 0.398 | 0.837 |
| AIDS | Yes | 0.035 | 0.216 | 0.271 | 0.440 |
| ARC | Yes | 0.009 | 1.230 | 0.311 | 1.773 |
| ARC | Yes | 0.036 | 0.931 | 0.292 | 0.909 |
| Asymptomatic | Yes | 1.852 | 2.707 | 0.279 | 0.532 |
| Asymptomatic | Yes | 2.190 | >3.0 | >3.0 | >3.0 |
| Asymptomatic | Yes | 0.139 | >3.0 | >3.0 | >3.0 |
| Asymptomatic | Yes | >3.0 | >3.0 | 2.542 | >3.0 |
| Asymptomatic | Yes | 0.365 | >3.0 | 1.352 | 2.874 |
| Hemophiliac | Yes | 0.054 | 0.970 | ND | 0.304 |
| Hemophiliac | Yes | 0.296 | 0.997 | ND | 0.041 |
| Hemophiliac | Yes | 0.558 | 0.504 | ND | 0.171 |
| Hemophiliac | Yes | 0.174 | 1.194 | ND | 0.407 |
| Hemophiliac | Yes | >3.0 | >3.0 | ND | 1.458 |
| Hemophiliac | Yes | 0.026 | 1.201 | ND | 0.079 |
| Hemophiliac | No | 0.037 | 0.053 | ND | 0.029 |
| Hemophiliac | No | 0.062 | 0.055 | ND | 0.023 |
| Hemophiliac | No | 0.010 | 0.006 | ND | 0.019 |
| High Risk | No | 0.057 | 0.027 | ND | 0.095 |
| High Risk | No | 0.051 | 0.011 | ND | 0.053 |
| Healthy Hetero. | No | 0.003 | 0.019 | 0.006 | 0.106 |
| Healthy Hetero. | No | 0.079 | 0.038 | 0.050 | 0.009 |
| Healthy Hetero. | No | 0.042 | 0.111 | 0.013 | 0.060 |
| Healthy Hetero. | No | 0.053 | 0.040 | 0.061 | 0.093 |

**Table 3**

| Patient Diagnosis | Confirmed Seropos. | Microtiter Saliva IgA | | | |
|---|---|---|---|---|---|
| | | p24 | gp41 | Peptide I | Peptide III |
| AIDS | Yes | 0.076 | 0.099 | 0.938 | 1.480 |
| AIDS | Yes | 0.035 | 0.065 | 0.559 | 1.165 |
| AIDS | Yes | 0.077 | 0.007 | 0.232 | 0.440 |
| AIDS | Yes | 0.064 | 0.040 | 0.982 | 1.251 |
| AIDS | Yes | 0.096 | 0.151 | 1.010 | 1.604 |
| AIDS | Yes | 0.056 | 0.355 | >3.0 | >3.0 |
| AIDS | Yes | 0.002 | 0.175 | 0.449 | 1.120 |
| AIDS | Yes | 0.069 | 0.040 | 0.933 | 1.738 |
| ARC | Yes | 0.040 | 0.239 | 2.845 | >3.0 |
| ARC | Yes | 0.094 | 0.172 | 2.036 | 2.836 |
| Asymptomatic | Yes | 0.144 | 0.078 | 0.840 | 1.054 |
| Asymptomatic | Yes | 0.419 | 0.110 | >3.0 | >3.0 |
| Asymptomatic | Yes | 0.288 | 0.773 | 1.635 | >3.0 |
| Asymptomatic | Yes | 0.146 | 0.186 | 2.424 | >3.0 |
| Asymptomatic | Yes | 0.634 | 0.678 | 1.544 | 2.988 |
| Hemophiliac | Yes | 0.045 | 0.020 | ND | >3.0 |
| Hemophiliac | Yes | 0.003 | 0.008 | ND | 2.520 |
| Hemophiliac | Yes | 0.174 | 0.093 | ND | 1.835 |
| Hemophiliac | Yes | 0.140 | 0.094 | ND | >3.0 |
| Hemophiliac | Yes | 0.280 | 0.435 | ND | >3.0 |
| Hemophiliac | Yes | 0.072 | 0.063 | ND | 2.947 |
| Hemophiliac | No | 0.061 | 0.016 | ND | 0.103 |
| Hemophiliac | No | 0.003 | 0.046 | ND | 0.011 |
| Hemophiliac | No | 0.044 | 0.008 | ND | 0.038 |
| High Risk | No | 0.083 | 0.007 | ND | 0.055 |
| High Risk | No | 0.094 | 0.056 | ND | 0.103 |
| Healthy Hetero. | No | 0.002 | 0.064 | 0.073 | 0.054 |
| Healthy Hetero. | No | 0.054 | 0.013 | 0.007 | 0.098 |
| Healthy Hetero. | No | 0.016 | 0.017 | 0.084 | 0.008 |
| Healthy Hetero. | No | 0.042 | 0.004 | 0.067 | 0.051 |

### Example 3

This example demonstrates a microparticle assay for detection of HIV antibodies utilizing Peptide III, recombinant DNA-derived p24 and recombinant DNA-derived gp41. One hundred microliters of amino-modified microparticles, 2.5% solids, 0.45 µm average diameter, commercially available from Polyscience, Warrington, Pennsylvania, were added to 1 ml of phosphate buffered saline (PBS) and 3.0 ml of sulfo-m-maleimido-benzoyl-N-hydroxysuccinimide (Sulfo-MBS) (1.0 mg/ml in PBS). The solution was stirred for 1 hour at room temperature after which the microparticles were isolated by centrifugation at 5000 x g speed, washed twice with PBS and resuspended in 1 ml PBS. Thirty microliters of a solution of Peptide III (1 mg/ml in distilled water) were added to the resuspended microparticles and stirred for 2 hours at room temperature. The microparticles were isolated by centrifugation at 5000 x g speed, washed twice with PBS containing 0.05% Tween® 20, and resuspended in PBS to yield a 0.125% solution. After resuspension in PBS, the particles were stored at 2-8°C for subsequent use in combination with p24 and gp41 coated microparticles in an assay for anti-HIV.

To make separate microparticle solutions of p24 coated microparticles and gp41 coated microparticles, one hundred microliters of amino-modified microparticles, 2.5% solids, 3.0 µm average diameter, commercially available from Polyscience, were added to 500 microliters of 50 mM N-methylmorpholine, pH 7.5 and 300 microliters of 2-iminothiolane (10 milligrams per milliliter in ice-cold 0.1 M sodium bicarbonate) for each antigen. The solution was incubated for 1 hour at room temperature. Two hundred microliters of the p24 antigen and gp41 antigen (described in Example 2 except that concentrations were as follows: p24, 654 µg/ml; gp41, 100,000 units/ml) were each incubated with 200 microliters of Sulfo-MBS (3.75 µg/ml in PBS) for 1 hour at room temperature. Each activated antigen was then added to the microparticles and incubated overnight at room temperature. The microparticles were isolated by centrifugation, washed twice with PBS containing 0.05% Tween® 20, and resuspended in PBS to yield a 0.125% solution. After resuspension in PBS, the particles were stored at 2-8°C for subsequent use in the microparticle assay for HIV antibodies described below.

Twenty microliters each of the antigen-coated microparticle solutions (Peptide III, p24 and gp41) were added dropwise to the center of a Whatman GF-D glass fiber filter arranged in a microparticle assay format. This assay format is described in more detail in copending U.S. Patent Application serial No. 831,013, filed on Feb. 18, 1986, assigned to the same assignee as that of the present invention and incorporated by reference herein. Three hundred microliters of an overcoat solution (10% bovine serum albumin, 3% sucrose, 0.05% Tween® 20 in PBS) were added and the unit incubated at 45° C for 90 minutes to dry the filter. It is to be noted that, in addition to the technique described in the foregoing examples, the antigens may be attached to the microparticles or other surfaces by a variety of methods, e.g., adsorption, use of specific antibodies, or the use of other various chemical activators.

A prefilter was situated above the unit, which contained the filter and the antigen-coated microparticles. Two hundred microliters of a 1:10 dilution of either serum or saliva in a sample diluent (10% bovine serum albumin, 2% Tween® 20 in PBS) were added to the prefilter. After the sample was absorbed, 200 microliters of an antibody-enzyme conjugate comprising a mixture of goat anti-human IgG and IgA:alkaline phosphatase were added to the matrix through the prefilter. After absorption, the prefilter was removed and 1 milliliter of a detergent wash solution (1M guanadine hydrochloride, 1M NaCl, .05% Tween® 20 in PBS) was added to the matrix to remove any excess antibody-enzyme conjugate. Then, 150 microliters of a chromogen indicator (bromo-chloro indolyl phosphate/nitro blue tetrazolium) were added to the matrix. After 2 minutes, 1 milliliter of the wash solution was added to the matrix. The matrix was checked visually. The appearance of a colored spot indicated that the specimen contained detectable levels of antibody to HIV. Samples tested by the foregoing procedure but not containing detectable levels of antibody to HIV produced no color in the matrix. The results are set forth in Tables 4 and 5.

**Table 4**

| Microparticle Assay | | | | |
|---|---|---|---|---|
| Patient Diagnosis | Confirmed Seropos. | Serum | | |
| | | p24 | gp41 | p24,gp41,PIII |
| AIDS | Yes | Neg | Pos | Pos |
| AIDS | Yes | Neg | Pos | Pos |
| AIDS | Yes | Neg | Pos | Pos |
| AIDS | Yes | Neg | Pos | Pos |
| AIDS | Yes | Neg | Pos | Pos |
| AIDS | Yes | Neg | Pos | Pos |
| AIDS | Yes | Neg | Pos | Pos |
| AIDS | Yes | Neg | Pos | Pos |
| ARC | Yes | Neg | Pos | Pos |
| ARC | Yes | Neg | Pos | Pos |
| Asymptomatic | Yes | Pos | Pos | Pos |
| Asymptomatic | Yes | Pos | Pos | Pos |
| Asymptomatic | Yes | Pos | Pos | Pos |
| Asymptomatic | Yes | Pos | Pos | Pos |
| Asymptomatic | Yes | Pos | Pos | Pos |
| Hemophiliac | Yes | Pos | Pos | Pos |
| Hemophiliac | Yes | Pos | Pos | Pos |
| Hemophiliac | Yes | Pos | Pos | Pos |
| Hemophiliac | Yes | Pos | Pos | Pos |
| Hemophiliac | Yes | Pos | Pos | Pos |
| Hemophiliac | Yes | Pos | Pos | Pos |
| Hemophiliac | No | Neg | Neg | Neg |
| Hemophiliac | No | Neg | Neg | Neg |
| Hemophiliac | No | Neg | Neg | Neg |
| High Risk | No | Neg | Neg | Neg |
| High Risk | No | Neg | Neg | Neg |
| Healthy Hetero. | No | Neg | Neg | Neg |
| Healthy Hetero. | No | Neg | Neg | Neg |
| Healthy Hetero. | No | Neg | Neg | Neg |
| Healthy Hetero. | No | Neg | Neg | Neg |

**Table 5**

| Microparticle Assay | | | | |
|---|---|---|---|---|
| Patient Diagnosis | Confirmed Seropos. | Saliva | | |
| | | p24 | gp41 | p24,gp41,PIII |
| AIDS | Yes | Neg | Pos | Pos |
| AIDS | Yes | Neg | Neg | Pos |
| AIDS | Yes | Neg | Pos | Pos |
| AIDS | Yes | Neg | Neg | Pos |
| AIDS | Yes | Neg | Pos | Pos |
| AIDS | Yes | Neg | Pos | Pos |
| AIDS | Yes | Neg | Pos | Pos |
| AIDS | Yes | Neg | Pos | Pos |
| ARC | Yes | Neg | Pos | Pos |
| ARC | Yes | Neg | Pos | Pos |
| Asymptomatic | Yes | Pos | Pos | Pos |
| Asymptomatic | Yes | Pos | Pos | Pos |
| Asymptomatic | Yes | Pos | Pos | Pos |
| Asymptomatic | Yes | Pos | Pos | Pos |
| Asymptomatic | Yes | Pos | Pos | Pos |
| Hemophiliac | Yes | Neg | Pos | Pos |
| Hemophiliac | Yes | Pos | Pos | Pos |
| Hemophiliac | Yes | Pos | Pos | Pos |
| Hemophiliac | Yes | Neg | Pos | Pos |
| Hemophiliac | Yes | Pos | Pos | Pos |
| Hemophiliac | Yes | Neg | Pos | Pos |
| Hemophiliac | No | Neg | Neg | Neg |
| Hemophiliac | No | Neg | Neg | Neg |
| Hemophiliac | No | Neg | Neg | Neg |
| High Risk | No | Neg | Neg | Neg |
| High Risk | No | Neg | Neg | Neg |
| Healthy Hetero. | No | Neg | Neg | Neg |
| Healthy Hetero. | No | Neg | Neg | Neg |
| Healthy Hetero. | No | Neg | Neg | Neg |
| Healthy Hetero. | No | Neg | Neg | Neg |

### Example 4

Peptides I, II or III or any combination of these peptides with one another or with other HIV antigens, may be employed to produce antisera or as a vaccine.

Antisera is specifically produced by immunizing rabbits with injections of Peptides I or III according to the present invention as follows. The peptide is coupled to a carrier protein thyroglobulin by the following general procedure: To a solution of the selected peptide (2 mg) in 1 ml of either distilled water or dimethylformamide, is added 36 µl of a solution of 1-ethyl-3 (3-dimethylamino propyl) carbodiimide (Sigma Chemical Co., St. Louis, Missouri, 7.0 mg/ml H₂O) at 0°C. The mixture is stirred for 5-10 minutes at 0°C. Next, 0.5 ml of thyroglobulin solution (Sigma, 10 mg/ml in PBS) is added to this reaction mixture and stirred overnight at 0°C. Finally, the mixture is dialyzed against PBS buffer with three changes of the buffer.

New Zealand white rabbits were inoculated with 250 mg of the conjugated peptide mixed (1:1) with complete Freund's adjuvant. All subsequent boosts contained conjugated Peptide mixed 1:1 with incomplete Freund's adjuvant. Animals were bled two weeks after each boost. Bleeds were processed to yield polyclonal antibodies in the serum. Peptide antibodies so generated immunoprecipitated gp160 and gp120 from ³⁵S-methionine and ³⁵S-cysteine labelled cell lysates. These antibodies may, for example, be utilized as reagents in a diagnostic assay, for affinity purification of gp120 or gp160 antigens or as a passive vaccine for therapeutic or prophylactic applications.

An active vaccine solution according to the present invention may be prepared by suspending Peptides I, II or III or a combination of these (or in combination with other HIV antigens) in an immunologically acceptable diluent, adjuvant or carrier. Initial and booster injections or oral delivery are used to confer immunity.

### Example 5

Monoclonal antibodies according to the present invention may be produced by injecting mice with immunizing doses of Peptides I, II or III or any combination of these with or without other epitopes of HIV. The peptide of interest is coupled to a carrier protein before injection as described in Example 4. The mouse spleens are then removed from the immunized animals and spleen cells are fused to myeloma cells (e.g. NS-1 cells) using polyethylene glycol. Hybridoma cells producing monoclonals are selected by screening in a suitable cell culture medium such as hypoxanthine aminoptern thymidine (HAT) medium. Monoclonal antibodies specific for HIV proteins may be isolated by affinity chromatography from media in which such hybridomas have been cultured.

The peptides of the invention have many advantages. First, they are noninfectious and therefore safer in diagnostic applications. Second, these peptides can be produced in large quantities at a low cost. Third, a diagnostic assay including these peptides alone, in combination with one another or in combination with other HIV epitopes is more sensitive and specific than previous HIV assays. Fourth, the peptides of the invention appear to have excellent application for saliva screening assays for HIV.

While specific examples have been given to illustrate the invention, it is to be understood that those skilled in the art will recognize variations which come within the scope of the invention as claimed. For example, the peptides of the invention may be used in conjunction with a number of HIV or other antigens or antibodies in diagnostic testing and in production of antibodies for vaccines or diagnostic assays.

## Claims (Claims for the following Contracting State(s): AT, BE, CH, DE, FR, GB, IT, LI, NL)

1. A peptide selected from the group consisting of: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;

(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X;

(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group).

2. The peptide of claim 1 wherein said peptide is made in combination with other epitopes of HIV as a fusion protein.

3. The peptide of claim 2 in combination with an HIV antigen selected from the group consisting of HIV p24, gp41, gp120 and combinations thereof.

4. An immunoassay for detection of HIV antibodies in a biological sample comprising:
a) coating a solid phase with a peptide selected from the group consisting of the peptides of Claim 1;
b) incubating the solid phase with the biological sample;
c) incubating the solid phase with an anti-human Ig labeled with a detectable label; and
d) detecting the label to determine the presence of anti-HIV in the sample.

5. The immunoassay of claim 4 wherein the solid phase is further coated in step (a) with an HIV antigen selected from the group consisting of p24, gp41 and gp120 and combinations thereof.

6. The immunoassay of claim 4 wherein the solid phase has properties independently selected from one or more of the following:
a) the solid phase in step (a) is coated with the peptide of claim 2;
b) the anti-human Ig is selected from the group consisting of anti-human IgG, IgA, IgM and mixtures thereof;
c) the detectable label is selected from the group consisting of enzymes, radioisotopes and fluorescent molecules.

7. An immunoassay for detection of HIV antibodies in human saliva test sample comprising:
a) coating a solid phase with a peptide selected from the group consisting of the peptides of Claim 1;
b) incubating the solid phase with the saliva test sample;
c) incubating the solid phase with an anti-human Ig labeled with a detectable label; and
d) detecting the label to determine the presence of anti-HIV antibodies in the saliva test sample.

8. The immunoassay of claim 7 wherein the solid phase has properties independently selected from one or more of the following:
a) the solid phase is further coated in step (a) with an HIV antigen selected from the group consisting of HIV p24, gp41 and gp120 and combinations thereof;
b) the peptide is Peptide III of Claim 1 and the HIV antigens are p24 and gp41;
c) the anti-human Ig is selected from the group consisting of anti-human IgG, IgA, IgM and mixtures thereof;
d) the detectable label is selected from the group consisting of enzymes, radioisotopes and fluorescent molecules.

9. An HIV antibody prepared by immunizing an animal with a peptide selected from the group consisting of the peptides of Claim 1 and mixtures thereof in a suitable adjuvant and obtaining antibodies from the sera of the animal.

10. The HIV antibody of claim 9 wherein the animal is immunized with the peptide in combination with at least one other HIV antigen.

11. A monoclonal antibody to HIV prepared by injecting an animal with an immunizing dose of a peptide selected from the group consisting of the peptides of Claim 1 and mixtures thereof, removing the spleen from the animal, fusing the spleen with myeloma cells, culturing the fused cells in a suitable culture medium, and collecting HIV antibodies from said culture.

12. The monoclonal antibody of claim 11 wherein the animal is immunized with the peptide in combination with at least one other HIV antigen.

13. An HIV vaccine prepared by suspending a peptide selected from the group consisting of the peptides of Claim 1 and mixtures thereof in a suitable pharmaceutically acceptable carrier.

14. The vaccine of claim 13 wherein the peptide is further combined with at least one other HIV antigen.

## Claims (Claims for the following Contracting State(s): ES)

1. An immunoassay method for detection of HIV antibodies in a biological sample comprising:
a) coating a solid phase with a peptide selected from the group consisting of
i) a peptide of the formula: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;
ii) a peptide of the formula: 
(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X;
iii) a peptide of the formula: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group);
b) incubating the solid phase with the biological sample;
c) incubating the solid phase with an anti-human Ig labeled with a detectable label; and
d) detecting the label to determine the presence of anti-HIV in the sample.

2. The immunoassay method of claim 1 wherein the solid phase is further coated in step (a) with an HIV antigen selected from the group consisting of p24, gp41 and gp120 and combinations thereof.

3. The immunoassay method of claim 1 wherein the solid phase has properties independently selected from one or more of the following:
a) the solid phase in step (a) is coated with a peptide which is a fusion protein of the peptide in step (a) in combination with other epitopes of HIV;
b) the anti-human Ig is selected from the group consisting of anti-human IgG, IgA, IgM and mixtures thereof;
c) the detectable label is selected from the group consisting of enzymes, radioisotopes and fluorescent molecules.

4. An immunoassay method for detection of HIV antibodies in human saliva test sample comprising:
a) coating a solid phase with a peptide selected from the group consisting of
i) a peptide of the formula: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;
ii) a peptide of the formula: 
(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X;
iii) a peptide of the formula: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group);
b) incubating the solid phase with the saliva test sample;
c) incubating the solid phase with an anti-human Ig labeled with a detectable label; and
d) detecting the label to determine the presence of anti-HIV antibodies in the saliva test sample.

5. The immunoassay method of claim 4 wherein the solid phase has properties independently selected from one or more of the following:
a) the solid phase is further coated in step (a) with an HIV antigen selected from the group consisting of p24, gp41 and gp120 and combinations thereof;
b) the peptide is Peptide III and the HIV antigens are p24 and gp41;
c) the anti-human Ig is selected from the group consisting of anti-human IgG, IgA, IgM and mixtures thereof;
d) the detectable label is selected from the group consisting of enzymes, radioisotopes and fluorescent molecules.

6. A method for preparing an HIV antibody comprising immunizing an animal with a peptide selected from the group consisting of
i) a peptide of the formula: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;
ii) a peptide of the formula: 
(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X;
iii) a peptide of the formula: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group), and mixtures thereof in a suitable adjuvant and obtaining antibodies from the sera of the animal.

7. The method of claim 6 wherein the animal is immunized with the peptide in combination with at least one other HIV antigen.

8. A method for preparing a monoclonal antibody to HIV comprising injecting an animal with an immunizing dose of a peptide selected from the group consisting of
i) a peptide of the formula: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;
ii) a peptide of the formula: 
(II) Y-Arg-Asp-Asn-Trp-Arg-Ser-Glu-Leu-Tyr-Lys-
     Tyr-Lys-Val-Ile-Lys-Ile-Glu-Pro-Leu-Gly-Ile-
     Ala-Pro-Thr-Lys-Ala-Lys-Arg-Arg-Val-Val-Gln-
     Arg-Glu-Lys-Arg-X;
iii) a peptide of the formula: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group), and mixtures thereof, removing the spleen from the animal, fusing the spleen with myeloma cells, culturing the fused cells in a suitable culture medium, and collecting HIV antibodies from said culture.

9. The method of claim 8 wherein the animal is immunized with the peptide in combination with at least one other HIV antigen.

10. A method for preparing an HIV vaccine comprising suspending a peptide selected from the group consisting of
i) a peptide of the formula: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;
ii) a peptide of the formula: 
(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X;
iii) a peptide of the formula: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group), and mixtures thereof in a suitable pharmaceutically acceptable carrier.

11. The method of Claim 10 wherein the peptide is further combined with at least one other HIV antigen.

12. The method according to Claim 6, 8 or 10 wherein said peptide is made in combination with other epitopes of HIV as a fusion protein.

13. The method according to Claim 7, 9 or 11 wherein said other HIV antigen is selected from the group consisting of HIV p24, gp 41, gp 120 and combinations thereof.

14. A process for preparing a peptide selected from the group consisting of
i) a peptide of the formula: 
(I) Y-Tyr-Lys-Tyr-Lys-Val-Ile-Lys-Ile-Glu-Pro-
Leu-Gly-Ile-Ala-Pro-Thr-Lys-Ala-Lys-Arg-
Arg-Val-Val-Gln-Arg-Glu-Lys-Arg-X;
ii) a peptide of the formula: 
(II) Y-Arg-Asp-Asn-Trp-Arg-Ser-Glu-Leu-Tyr-Lys-
Tyr-Lys-Val-Ile-Lys-Ile-Glu-Pro-Leu-Gly-Ile-
Ala-Pro-Thr-Lys-Ala-Lys-Arg-Arg-Val-Val-Gln-
Arg-Glu-Lys-Arg-X;
iii) a peptide of the formula: 
(III) Y-Thr-Arg-Asp-Gly-Gly-Asn-Ser-Asn-Glu-Ser-
Glu-Ile-Phe-Arg-Pro-Gly-Gly-Gly-Asp-Met-Arg-
Asp-Asn-Trp-Arg-Ser-Glu-Leu-Tyr-Lys-Tyr-Lys-
Val-Val-Lys-Ile-Glu-Pro-Leu-Gly-Val-Ala-Pro-
Thr-Lys-Ala-Lys-Arg-Arg-Val-Val-Gln-Arg-Glu-
Lys-Arg-X;
 and substitution, deletion and addition analogs thereof, wherein Y corresponds to -H or an amino acid, and X is -OH, -NH₂ or -NR₁R₂ (wherein R is an alkyl group), wherein said process comprises preparing said peptides either
a) in solution or by solid phase peptide methodology using stepwise or fragment coupling protocols;
b) enzymatically;
c) by synthesizing, cloning and expressing regions of genes coding for said peptides using recombinant DNA methodology.

15. A process according to Claim 14, part (c), wherein the genes are subcloned and expressed in E. coli, yeast or mammalian cells.

16. A process according to Claim 14 wherein the peptides are combined with other epitopes or antigens of HIV as fusion products.

## Patentansprüche (Patentansprüche für folgende(n) Vertragsstaat(en): AT, BE, CH, DE, FR, GB, IT, LI, NL)

1. Ein Peptid, ausgewählt aus der Gruppe bestehend aus 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;

(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
Val-Val-Gln-Arg-Glu-Lys-Arg-X;

(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanaloge hiervon, in denen Y -H oder eine Aminosäure und X -OH, -NH₂ oder -NR₁R₂ (worin R eine Alkylgruppe ist) bedeuten.

2. Das Peptid des Anspruchs 1, worin das genannte Peptid in Kombination mit anderen Antigen-Determinanten von HIV als ein Fusionsprotein erzeugt wird.

3. Das Peptid des Anspruchs 2 in Kombination mit einem HIV-Antigen, ausgewählt aus der Gruppe, die aus HIV p24, gp41, gp120 und Kombinationen hiervon besteht.

4. Ein Immunoassay für den Nachweis von HIV-Antikörpern in einer biologischen Probe, umfassend:
a) Beschichten einer festen Phase mit einem Peptid, ausgewählt aus der Gruppe, die aus den Peptiden des Anspruchs 1 besteht;
b) Inkubieren der festen Phase mit der biologischen Probe;
c) Inkubieren der festen Phase mit einem anti-human Ig, das mit einer nachweisbaren Kennzeichnung markiert ist; und
d) Nachweis der Kennzeichnung, um die Anwesenheit von Anti-HIV in der Probe zu bestimmen.

5. Der Immunoassay des Anspruchs 4, worin die feste Phase in der Stufe a) außerdem mit einem HIV-Antigen, ausgewählt aus der Gruppe, welche aus p24, gp41 und gp120 und Kombinationen hiervon besteht, beschichtet wird.

6. Der Immunoassay des Anspruchs 4, worin die feste Phase Eigenschaften aufweist, welche unabhängig voneinander aus einer oder mehreren der folgenden ausgewählt sind:
a) Die feste Phase der Stufe (a) wird mit dem Peptid des Anspruchs 2 beschichtet;
b) das anti-human Ig ist aus der Gruppe ausgewählt, die aus anti-human IgG, IgA, IgM und Mischungen hiervon besteht;
c) die nachweisbare Kennzeichnung ist aus der Gruppe ausgewählt, die aus Enzymen, radioaktiven Isotopen und fluoreszierenden Molekülen besteht.

7. Ein Immunoassay für den Nachweis von HIV-Antikörpern in einer Probe von humanem Speichel, umfassend:
a) Beschichten einer festen Phase mit einem Peptid, ausgewählt aus der Gruppe der Peptide des Anspruchs 1;
b) Inkubieren der festen Phase mit der Speichelprobe;
c) Inkubieren der festen Phase mit einem anti-human Ig, das mit einer nachweisbaren Kennzeichnung markiert ist; und
d) Nachweis der Kennzeichnung, um die Anwesenheit von Anti-HIV-Antikörpern in der Speichelprobe zu bestimmen.

8. Der Immunoassay des Anspruchs 7, worin die feste Phase Eigenschaften aufweist, welche unabhängig voneinander aus einer oder mehreren der folgenden ausgewählt sind:
a) Die feste Phase wird in der Stufe (a) außerdem mit einem HIV-Antigen beschichtet, das aus der Gruppe ausgewählt ist, welche aus p24, gp41 und gp120 und Kombinationen hiervon besteht;
b) das Peptid ist das Peptid III des Anspruchs 1 und die HIV-Antigene sind p24 und gp41;
c) das anti-human Ig ist aus der Gruppe ausgewählt, die aus anti-human IgG, IgA, IgM und Mischungen hiervon besteht;
d) die nachweisbare Kennzeichnung ist aus der Gruppe ausgewählt, die aus Enzymen, radioaktiven Isotopen und fluoreszierenden Molekülen besteht.

9. Ein HIV-Antikörper, hergestellt durch Immunisierung eines Tieres mit einem Peptid, das ausgewählt ist aus der Gruppe der Peptide des Anspruchs 1 und deren Mischungen, in einem geeigneten Adjuvans und Gewinnung der Antikörper aus den Seren des Tieres.

10. Der HIV-Antikörper des Anspruchs 9, worin das Tier mit dem Peptid in Kombination mit zumindest einem anderen HIV-Antigen immunisiert wird.

11. Ein monoklonaler Antikörper gegen HIV, hergestellt, indem einem Tier eine immunisierende Dosis eines Peptids, das ausgewählt ist aus der Gruppe der Peptide des Anspruchs 1 und Mischungen hiervon, injiziert wird, die Milz des Tieres entnommen wird, die Milz mit Myelomzellen fusioniert wird, die fusionierten Zellen in einem geeigneten Kulturmedium kultiviert werden und die HIV-Antikörper aus der genannten Kultur aufgesammelt werden.

12. Der monoklonale Antikörper des Anspruchs 11, worin das Tier mit dem Peptid in Kombination mit zumindest einem anderen HIV-Antigen immunisiert wird.

13. Eine HIV-Vakzine, hergestellt durch Suspendieren eines Peptids, ausgewählt aus der Gruppe der Peptide des Anspruchs 1 und Mischungen hiervon, in einem geeigneten pharmazeutisch annehmbaren Träger.

14. Die Vakzine des Anspruchs 13, wobei das Peptid ferner mit zumindest einem anderen HIV-Antigen kombiniert wird.

## Patentansprüche (Patentansprüche für folgende(n) Vertragsstaat(en): ES)

1. Ein Immunoassayverfahren für den Nachweis von HIV-Antikörpern in einer biologischen Probe, umfassend:
a) Beschichten einer festen Phase mit einem Peptid, ausgewählt aus der Gruppe bestehend aus
i) einem Peptid der Formel 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;
ii) einem Peptid der Formel 
(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
Val-Val-Gln-Arg-Glu-Lys-Arg-X;
iii) einem Peptid der Formel 
(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanalogen hiervon, in denen Y -H oder eine Aminosäure und X -OH, -NH₂ oder -NR₁R₂ (worin R eine Alkylgruppe ist) bedeuten;
b) Inkubieren der festen Phase mit der biologischen Probe;
c) Inkubieren der festen Phase mit einem anti-human Ig, das mit einer nachweisbaren Kennzeichnung markiert ist; und
d) Nachweis der Kennzeichnung, um die Anwesenheit von Anti-HIV in der Probe zu bestimmen.

2. Das Immunoassayverfahren des Anspruchs 1, worin die feste Phase in der Stufe a) außerdem mit einem HIV-Antigen, ausgewählt aus der Gruppe, welche aus p24, gp41 und gp120 und Kombinationen hiervon besteht, beschichtet wird.

3. Das Immunoassayverfahren des Anspruchs 1, worin die feste Phase Eigenschaften aufweist, welche unabhängig voneinander aus einer oder mehreren der folgenden ausgewählt sind:
a) Die feste Phase der Stufe (a) wird mit einem Peptid beschichtet, welches ein Fusionsprotein des Peptids in der Stufe (a) in Kombination mit anderen Antigen-Determinanten von HIV ist;
b) das anti-human Ig ist aus der Gruppe ausgewählt, die aus anti-human IgG, IgA, IgM und Mischungen hiervon besteht;
c) die nachweisbare Kennzeichnung ist aus der Gruppe ausgewählt, die aus Enzymen, radioaktiven Isotopen und fluoreszierenden Molekülen besteht.

4. Ein Immunoassayverfahren für den Nachweis von HIV-Antikörpern in einer Probe von humanem Speichel, umfassend:
a) Beschichten einer festen Phase mit einem Peptid, ausgewählt aus der Gruppe bestehend aus
i) einem Peptid der Formel 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;
ii) einem Peptid der Formel 
(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
Val-Val-Gln-Arg-Glu-Lys-Arg-X;
iii) einem Peptid der Formel 
(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanalogen hiervon, in denen Y -H oder eine Aminosäure und X -OH, -NH₂ oder -NR₁R₂ (worin R eine
Alkylgruppe ist) bedeuten;
b) Inkubieren der festen Phase mit der Speichelprobe;
c) Inkubieren der festen Phase mit einem anti-human Ig, das mit einer nachweisbaren Kennzeichnung markiert ist; und
d) Nachweis der Kennzeichnung, um die Anwesenheit von Anti-HIV-Antikörpern in der Speichelprobe zu bestimmen.

5. Das Immunoassayverfahren des Anspruchs 4, worin die feste Phase Eigenschaften aufweist, welche unabhängig voneinander aus einer oder mehreren der folgenden ausgewählt sind:
a) Die feste Phase wird in der Stufe (a) außerdem mit einem HIV-Antigen beschichtet, das aus der Gruppe ausgewählt ist, welche aus p24, gp41 und gp120 und Kombinationen hiervon besteht;
b) das Peptid ist das Peptid III und die HIV-Antigene sind p24 und gp41;
c) das anti-human Ig ist aus der Gruppe ausgewählt, die aus anti-human IgG, IgA, IgM und Mischungen hiervon besteht;
d) die nachweisbare Kennzeichnung ist aus der Gruppe ausgewählt, die aus Enzymen, radioaktiven Isotopen und fluoreszierenden Molekülen besteht.

6. Ein Verfahren zur Herstellung eines HIV-Antikörpers, umfassend die Immunisierung eines Tieres mit einem Peptid, das ausgewählt ist aus der Gruppe bestehend aus
i) einem Peptid der Formel 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;
ii) einem Peptid der Formel 
(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
Val-Val-Gln-Arg-Glu-Lys-Arg-X;
iii) einem Peptid der Formel 
(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanalogen hiervon, in denen Y -H oder eine Aminosäure und X -OH, -NH₂ oder -NR₁R₂ (worin R eine Alkylgruppe ist) bedeuten, und Mischungen hiervon in einem geeigneten Adjuvans und Gewinnung der Antikörper aus den Seren des Tieres.

7. Das Verfahren des Anspruchs 6, worin das Tier mit dem Peptid in Kombination mit zumindest einem anderen HIV-Antigen immunisiert wird.

8. Ein Verfahren zur Herstellung eines monoklonalen Antikörpers gegen HIV, umfassend: Injizieren einer immunisierenden Dosis eines Peptids, das ausgewählt ist aus der Gruppe bestehend aus
i) einem Peptid der Formel 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;
ii) einem Peptid der Formel 
(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
 Val-Val-Gln-Arg-Glu-Lys-Arg-X;
iii) einem Peptid der Formel 
(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanalogen hiervon, in denen Y -H oder eine Aminosäure und X -OH, -NH₂ oder -NR₁R₂ (worin R eine Alkylgruppe ist) bedeuten, und Mischungen hiervon, einem Tier, Entnehmen der Milz des Tieres, Fusionieren der Milz mit Myelomzellen, Kultivieren der fusionierten Zellen in einem geeigneten Kulturmedium und Aufsammeln der HIV-Antikörper aus der genannten Kultur.

9. Das Verfahren des Anspruchs 8, worin das Tier mit dem Peptid in Kombination mit zumindest einem anderen HIV-Antigen immunisiert wird.

10. Ein Verfahren zur Herstellung einer HIV-Vakzine, umfassend: Suspendieren eines Peptids, ausgewählt aus der Gruppe bestehend aus
i) einem Peptid der Formel 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;
ii) einem Peptid der Formel 
(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
Val-Val-Gln-Arg-Glu-Lys-Arg-X;
iii) einem Peptid der Formel 
(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanalogen hiervon, in denen Y -H oder eine Aminosäure und X -OH, -NH₂ oder -NR₁R₂ (worin R eine Alkylgruppe ist) bedeuten, und aus Mischungen hiervon, in einem geeigneten pharmazeutisch annehmbaren Träger.

11. Das Verfahren des Anspruchs 10, worin das Peptid außerdem mit zumindest einem anderen HIV-Antigen kombiniert wird.

12. Das Verfahren gemäß den Ansprüchen 6, 8 oder 10, worin das genannte Peptid in Kombination mit anderen Antigen-Determinanten von HIV als Fusionsprotein erzeugt wird.

13. Das Verfahren gemäß den Ansprüchen 7, 9 oder 11, worin das genannte andere HIV-Antigen aus der Gruppe ausgewählt ist, welche aus HIV p24, gp41, gp 120 und Kombinationen hiervon besteht.

14. Ein Verfahren zur Herstellung eines Peptids, ausgewählt aus der Gruppe bestehend aus
i) einem Peptid der Formel 
(I) Y-Tyr-Lys-Tyr-Lys-Val-Ile-Lys-Ile-Glu-Pro-Leu-Gly-Ile-Ala-
Pro-Thr-Lys-Ala-Lys-Arg-Arg-Val-Val-Gln-Arg-Glu-Lys-Arg-X;
ii) einem Peptid der Formel 
(II) Y-Arg-Asp-Asn-Trp-Arg-Ser-Glu-Leu-Tyr-Lys-Tyr-Lys-Val-Ile-
Lys-Ile-Glu-Pro-Leu-Gly-Ile-Ala-Pro-Thr-Lys-Ala-Lys-Arg-Arg-
Val-Val-Gln-Arg-Glu-Lys-Arg-X;
iii) einem Peptid der Formel 
(III) Y-Thr-Arg-Asp-Gly-Gly-Asn-Ser-Asn-Glu-Ser-Glu-Ile-Phe-Arg-
Pro-Gly-Gly-Gly-Asp-Met-Arg-Asp-Asn-Trp-Arg-Ser-Glu-Leu-Tyr-
Lys-Tyr-Lys-Val-Val-Lys-Ile-Glu-Pro-Leu-Gly-Val-Ala-Pro-Thr-
Lys-Ala-Lys-Arg-Arg-Val-Val-Gln-Arg-Glu-Lys-Arg-X;
 und Substitutions-, Deletions- und Additionsanalogen hiervon, in denen Y -H oder eine Aminosäure und X -OH, -NH₂ oder -NR₁R₂ (worin R eine Alkylgruppe ist) bedeuten, worin das genannte Verfahren die Herstellung der genannten Peptide
a) in Lösung oder mittels der Festphasen-Peptidmethodik unter Verwendung von stufenweisen oder Fragmentkupplungs-Protokollen;
b) enzymatisch;
c) durch Synthetisieren, Klonen und Exprimieren von Genregionen, die für die genannten Peptide kodieren, unter Verwendung der rekombinanten DNA-Methodik; umfaßt.

15. Ein Verfahren gemäß Anspruch 14, Teil (c), worin die Gene in E. coli, Hefen oder Säugetierzellen subkloniert und exprimiert werden.

16. Ein Verfahren gemäß Anspruch 14, worin die Peptide mit anderen Antigen-Determinanten oder Antigenen von HIV als Fusionsprodukte kombiniert sind.

## Revendications (Revendications pour l'(les) Etat(s) contractant(s) suivant(s): AT, BE, CH, DE, FR, GB, IT, LI, NL)

1. Peptide choisi dans le groupe consistant en : 
(I)	Y–Tyr–Lys–Tyr–Lys–Val–Ile–Lys–Ile–Glu–Pro–Leu–
Gly–Ile–Ala–Pro–Thr–Lys–Ala–Lys–Arg–Arg–Val–Val–
Gln–Arg–Glu–Lys–Arg–X ;
(II)	Y–Arg–Asp–Asn–Trp–Arg–Ser–Glu–Leu–Tyr–Lys–Tyr–
Lys–Val–Ile–Lys–Ile–Glu–Pro–Leu–Gly–Ile–Ala–Pro–
Thr–Lys–Ala–Lys–Arg–Arg–Val–Val–Gln–Arg–Glu–
Lys–Arg–X ;
(III)	Y–Thr–Arg–Asp–Gly–Gly–Asn–Ser–Asn–Glu–Ser–Glu–
Ile–Phe–Arg–Pro–Gly–Gly–Gly–Asp–Met–Arg–Asp–
Asn–Trp–Arg–Ser–Glu–Leu–Tyr–Lys–Tyr–Lys–Val–
Val–Lys–Ile–Glu–Pro–Leu–Gly–Val–Ala–Pro–Thr–Lys–
Ala–Lys–Arg–Arg–Val–Val–Gln–Arg–Glu–Lys–Arg–X ;
 et ses analogues par substitution, délétion et addition, où Y correspond à -H ou un acide aminé, et X est -OH, -NH₂ ou -NR₁R₂ (R étant un groupe alkyle).

2. Peptide selon la revendication 1, ledit peptide étant préparé en combinaison avec d'autres épitopes de HIV sous forme d'une protéine de fusion.

3. Peptide selon la revendication 2 en combinaison avec un antigène de HIV choisi dans le groupe constituant en p24, gp41, gp120 de HIV et leurs combinaisons.

4. Immunodosage pour la détection d'anticorps contre HIV dans un échantillon biologique, comprenant :
a) le recouvrement d'une phase solide avec un peptide choisi dans le groupe consistant en les peptides de la revendication 1 ;
b) l'incubation de la phase solide avec l'échantillon biologique ;
c) l'incubation de la phase solide avec une Ig anti-humaine marquée avec un marqueur détectable ; et
d) la détection du marqueur pour déterminer la présence d'anti-HIV dans l'échantillon.

5. Immunodosage selon la revendication 4, dans lequel la phase solide est recouverte en outre dans l'étape (a) avec un antigène de HIV choisi dans le groupe consistant en p24, gp41 et gp120 et leurs combinaisons.

6. Immunodosage selon la revendication 4, dans lequel la phase solide a des propriétés choisies indépendamment parmi une ou plusieurs des suivantes :
a) la phase solide dans l'étape (a) est recouverte avec le peptide de la revendication 2 ;
b) l'Ig anti-humaine est choisie dans le groupe consistant en les IgG, IgA, IgM anti-humaines et leurs mélanges ;
c) le marqueur détectable est choisi dans le groupe consistant en les enzymes, les radioisotopes et les molécules fluorescentes.

7. Immunodosage pour la détection d'anticorps anti-HIV dans un échantillon à tester de salive humaine, comprenant :
a) le recouvrement d'une phase solide avec un peptide choisi dans le groupe consistant en les peptides de la revendication 1 ;
b) l'incubation de la phase solide avec l'échantillon à tester de salive ;
c) l'incubation de la phase solide avec une Ig anti-humaine marquée avec un marqueur détectable ; et
d) la détection du marqueur pour déterminer la présence d'anticorps anti-HIV dans l'échantillon à tester de salive.

8. Immunodosage selon la revendication 7, dans lequel la phase solide a des propriétés choisies indépendamment parmi une ou plusieurs des suivantes :
a) la phase solide est recouverte en outre dans l'étape (a) avec un antigène de HIV choisi dans le groupe consistant en p24, gp41 et gp120 de HIV et leurs combinaisons ;
b) le peptide est le peptide III de la revendication 1 et les antigènes de HIV sont p24 et gp41 ;
c) l'Ig anti-humaine est choisie dans le groupe consistant en les IgG, IgA, IgM anti-humaines et leurs mélanges ;
d) le marqueur détectable est choisi dans le groupe consistant en les enzymes, les radioisotopes et les molécules fluorescentes.

9. Anticorps anti-HIV préparé par immunisation d'un animal avec un peptide choisi dans le groupe consistant en les peptides de la revendication 1 et leurs mélanges dans un adjuvant approprié et par obtention d'anticorps à partir du sérum de l'animal.

10. Anticorps anti-HIV selon la revendication 9, dans lequel l'animal est immunisé avec le peptide en combinaison avec au moins un autre antigène de HIV.

11. Anticorps monoclonal dirigé contre HIV, préparé par injection à un animal d'une dose immunisante d'un peptide choisi dans le groupe consistant en les peptides de la revendication 1 et leurs mélanges, retrait de la rate de l'animal, fusion de la rate avec des cellules de myélome, culture des cellules fusionnées dans un milieu de culture approprié et récupération d'anticorps anti-HIV à partir de ladite culture.

12. Anticorps monoclonal selon la revendication 11, dans lequel l'animal est immunisé avec le peptide en combinaison avec au moins un autre antigène de HIV.

13. Vaccin contre HIV préparé par mise en suspension d'un peptide choisi dans le groupe consistant en les peptides de la revendication 1 et leurs mélanges dans un véhicule pharmaceutiquement acceptable approprié.

14. Vaccin selon la revendication 13, dans lequel le peptide est combiné en outre avec au moins un autre antigène de HIV.

## Revendications (Revendications pour l'(les) Etat(s) contractant(s) suivant(s): ES)

1. Procédé d'immunodosage pour la détection d'anticorps anti-HIV dans un échantillon biologique, comprenant :
a) le recouvrement d'une phase solide avec un peptide choisi dans le groupe consistant en ;
i) un peptide de formule : 
(I) Y-Tyr-Lys-Tyr-Lys-Val-Ile-Lys-Ile-Glu-Pro-Leu-
Gly-Ile-Ala-Pro-Thr-Lys-Ala-Lys-Arg-Arg-Val-Val-
Gln-Arg-Glu-Lys-Arg-X ;
ii) un peptide de formule : 
(II) Y-Arg-Asp-Asn-Trp-Arg-Ser-Glu-Leu-Tyr-Lys-Tyr-
Lys-Val-Ile-Lys-Ile-Glu-Pro-Leu-Gly-Ile-Ala-Pro-
Thr-Lys-Ala-Lys-Arg-Arg-Val-Val-Gln-Arg-Glu-
Lys-Arg-X ;
iii) un peptide de formule : 
(III) Y–Thr–Arg–Asp–Gly–Gly–Asn–Ser–Asn–Glu–Ser–Glu–
Ile–Phe–Arg–Pro–Gly–Gly–Gly–Asp–Met–Arg–Asp–
Asn–Trp–Arg–Ser–Glu–Leu–Tyr–Lys–Tyr–Lys–Val–
Val–Lys–Ile–Glu–Pro–Leu–Gly–Val–Ala–Pro–Thr–Lys–
Ala–Lys–Arg–Arg–Val–Val–Gln–Arg–Glu–Lys–Arg–X ;
 et ses analogues par substitution, délétion et addition, où Y correspond à -H ou un acide aminé, et X est -OH, -NH₂ ou -NR₁R₂ (R étant un groupe alkyle);
b) l'incubation de la phase solide avec l'échantillon biologique ;
c) l'incubation de la phase solide avec une Ig anti-humaine marquée avec un marqueur détectable ; et
d) la détection du marqueur pour déterminer la présence d'anti-HIV dans l'échantillon.

2. Procédé d'immunodosage selon la revendication 1, dans lequel la phase solide est recouverte en outre dans l'étape (a) avec un antigène de HIV choisi dans le groupe consistant en p24, gp41 et gp120 et leurs combinaisons.

3. Procédé d'immunodosage selon la revendication 1, dans lequel la phase solide a des propriétés choisies indépendamment parmi une ou plusieurs des suivantes :
a) la phase solide dans l'étape (a) est recouverte avec un peptide qui est une protéine de fusion du peptide de l'étape (a) en combinaison avec d'autres épitopes de HIV ;
b) l'Ig anti-humaine est choisie dans le groupe consistant en les IgG, IgA, IgM, anti-humaines et leurs mélanges ;
c) le marqueur détectable est choisi dans le groupe consistant en les enzymes, les radioisotopes et les molécules fluorescentes.

4. Procédé d'immunodosage pour la détection d'anticorps anti-HIV dans un échantillon à tester de salive humaine, comprenant :
a) le recouvrement d'une phase solide avec un peptide choisi dans le groupe consistant en ;
i) un peptide de formule : 
(I) Y-Tyr-Lys-Tyr-Lys-Val-Ile-Lys-Ile-Glu-Pro-Leu-
Gly-Ile-Ala-Pro-Thr-Lys-Ala-Lys-Arg-Arg-Val-Val-
Gln-Arg-Glu-Lys-Arg-X ;
ii) un peptide de formule : 
(II) Y-Arg-Asp-Asn-Trp-Arg-Ser-Glu-Leu-Tyr-Lys-Tyr-
Lys-Val-Ile-Lys-Ile-Glu-Pro-Leu-Gly-Ile-Ala-Pro-
Thr-Lys-Ala-Lys-Arg-Arg-Val-Val-Gln-Arg-Glu-
Lys-Arg-X ;
iii) un peptide de formule : 
(III) Y-Thr-Arg-Asp-Gly-Gly-Asn-Ser-Asn-Glu-Ser-Glu-
Ile-Phe-Arg-Pro-Gly-Gly-Gly-Asp-Met-Arg-Asp-
Asn-Trp-Arg-Ser-Glu-Leu-Tyr-Lys-Tyr-Lys-Val-
Val-Lys-Ile-Glu-Pro-Leu-Gly-Val-Ala-Pro-Thr-Lys-
Ala-Lys-Arg-Arg-Val-Val-Gln-Arg-Glu-Lys-Arg-X ;
 et ses analogues par substitution, délétion et addition, où Y correspond à -H ou un acide aminé, et X est -OH, -NH₂ ou -NR₁R₂ (R étant un groupe alkyle);
b) l'incubation de la phase solide avec l'échantillon à tester de salive ;
c) l'incubation de la phase solide avec une Ig anti-humaine marquée avec un marqueur détectable ; et
d) la détection du marqueur pour déterminer la présence d'anticorps anti-HIV dans l'échantillon à tester de salive.

5. Procédé d'immunodosage selon la revendication 4, dans lequel la phase solide a des propriétés choisies indépendamment parmi une ou plusieurs des suivantes :
a) la phase solide est recouverte en outre dans l'étape (a) avec un antigène de HIV choisi dans le groupe consistant en p24, gp41 et gp120 et leurs combinaisons ;
b) le peptide est le peptide III et les antigènes de HIV sont p24 et gp41 ;
c) l'Ig anti-humaine est choisie dans le groupe consistant en les IgG, IgA, IgM anti-humaines et leurs mélanges ;
d) le marqueur détectable est choisi dans le groupe consistant en les enzymes, les radioisotopes et les molécules fluorescentes.

6. Procédé de préparation d'un anticorps anti-HIV comprenant l'immunisation d'un animal avec un peptide choisi dans le groupe consistant en :
i) un peptide de formule : 
(I) Y-Tyr-Lys-Tyr-Lys-Val-Ile-Lys-Ile-Glu-Pro-Leu-
Gly-Ile-Ala-Pro-Thr-Lys-Ala-Lys-Arg-Arg-Val-Val-
Gln-Arg-Glu-Lys-Arg-X ;
ii) un peptide de formule : 
(II) Y-Arg-Asp-Asn-Trp-Arg-Ser-Glu-Leu-Tyr-Lys-Tyr-
Lys-Val-Ile-Lys-Ile-Glu-Pro-Leu-Gly-Ile-Ala-Pro-
Thr-Lys-Ala-Lys-Arg-Arg-Val-Val-Gln-Arg-Glu-
Lys-Arg-X ;
iii) un peptide de formule : 
(III) Y–Thr–Arg–Asp–Gly–Gly–Asn–Ser–Asn–Glu–Ser–Glu–
Ile–Phe–Arg–Pro–Gly–Gly–Gly–Asp–Met–Arg–Asp–
Asn–Trp–Arg–Ser–Glu–Leu–Tyr–Lys–Tyr–Lys–Val–
Val–Lys–Ile–Glu–Pro–Leu–Gly–Val–Ala–Pro–Thr–Lys–
Ala–Lys–Arg–Arg–Val–Val–Gln–Arg–Glu–Lys–Arg–X ;
 et ses analogues par substitution, délétion et addition, où Y correspond à -H ou un acide aminé, et X est -OH, -NH₂ ou -NR₁R₂ (R étant un groupe alkyle), et leurs mélanges dans un adjuvant approprié et l'obtention d'anticorps à partir du sérum de l'animal.

7. Procédé selon la revendication 6, dans lequel l'animal est immunisé avec le peptide en combinaison avec au moins un autre antigène de HIV.

8. Procédé de préparation d'un anticorps monoclonal dirigé contre HIV, comprenant l'injection à un animal d'une dose immunisante d'un peptide choisi dans le groupe consistant en :
i) un peptide de formule : 
(I) Y-Tyr-Lys-Tyr-Lys-Val-Ile-Lys-Ile-Glu-Pro-Leu-
Gly-Ile-Ala-Pro-Thr-Lys-Ala-Lys-Arg-Arg-Val-Val-
Gln-Arg-Glu-Lys-Arg-X ;
ii) un peptide de formule : 
(II) Y–Arg–Asp–Asn–Trp–Arg–Ser–Glu–Leu–Tyr–Lys–Tyr–
Lys–Val–Ile–Lys–Ile–Glu–Pro–Leu–Gly–Ile–Ala–Pro–
Thr–Lys–Ala–Lys–Arg–Arg–Val–Val–Gln–Arg–Glu–
Lys–Arg–X ;
iii) un peptide de formule : 
(III) Y–Thr–Arg–Asp–Gly–Gly–Asn–Ser–Asn–Glu–Ser–Glu–
Ile–Phe–Arg–Pro–Gly–Gly–Gly–Asp–Met–Arg–Asp–
Asn–Trp–Arg–Ser–Glu–Leu–Tyr–Lys–Tyr–Lys–Val–
Val–Lys–Ile–Glu–Pro–Leu–Gly–Val–Ala–Pro–Thr–Lys–
Ala–Lys–Arg–Arg–Val–Val–Gln–Arg–Glu–Lys–Arg–X ;
 et ses analogues par substitution, délétion et addition, où Y correspond à -H ou un acide aminé, et X est -OH, -NH₂ ou -NR₁R₂ (R étant un groupe alkyle), et leurs mélanges, le retrait de la rate de l'animal, la fusion de la rate avec des cellules de myélome, la culture des cellules fusionnées dans un milieu de culture approprié et la récupération d'anticorps anti-HIV à partir de ladite culture.

9. Procédé selon la revendication 8, dans lequel l'animal est immunisé avec le peptide en combinaison avec au moins un autre antigène de HIV.

10. Procédé de préparation d'un vaccin contre HIV, comprenant la mise en suspension d'un peptide choisi dans le groupe consistant en :
i) un peptide de formule : 
(I) Y-Tyr-Lys-Tyr-Lys-Val-Ile-Lys-Ile-Glu-Pro-Leu-
Gly-Ile-Ala-Pro-Thr-Lys-Ala-Lys-Arg-Arg-Val-Val-
Gln-Arg-Glu-Lys-Arg-X ;
ii) un peptide de formule : 
(II) Y–Arg–Asp–Asn–Trp–Arg–Ser–Glu–Leu–Tyr–Lys–Tyr–
Lys–Val–Ile–Lys–Ile–Glu–Pro–Leu–Gly–Ile–Ala–Pro–
Thr–Lys–Ala–Lys–Arg–Arg–Val–Val–Gln–Arg–Glu–
Lys–Arg–X ;
iii) un peptide de formule : 
(III) Y-Thr-Arg-Asp-Gly-Gly-Asn-Ser-Asn-Glu-Ser-Glu-
Ile-Phe-Arg-Pro-Gly-Gly-Gly-Asp-Met-Arg-Asp-
Asn-Trp-Arg-Ser-Glu-Leu-Tyr-Lys-Tyr-Lys-Val-
Val-Lys-Ile-Glu-Pro-Leu-Gly-Val-Ala-Pro-Thr-Lys-
Ala-Lys-Arg-Arg-Val-Val-Gln-Arg-Glu-Lys-Arg-X ;
 et ses analogues par substitution, délétion et addition, où Y correspond à -H ou un acide aminé, et X est -OH, -NH₂ ou -NR₁R₂ (R étant un groupe alkyle), et leurs mélanges dans un véhicule pharmaceutiquement acceptable approprié.

11. Procédé selon la revendication 10, dans lequel le peptide est combiné en outre avec au moins un autre antigène de HIV.

12. Procédé selon la revendication 6, 8 ou 10, dans lequel ledit peptide est préparé en combinaison avec d'autres épitopes de HIV sous forme d'une protéine de fusion.

13. Procédé selon la revendication 7, 9 ou 11, dans lequel ledit autre antigène de HIV est choisi dans le groupe consistant en p24, gp41, gp120 de HIV et leurs combinaisons.

14. Procédé de préparation d'un peptide choisi dans le groupe consistant en
i) un peptide de formule : 
(I) Y-Tyr-Lys-Tyr-Lys-Val-Ile-Lys-Ile-Glu-Pro-Leu-
Gly-Ile-Ala-Pro-Thr-Lys-Ala-Lys-Arg-Arg-Val-Val-
Gln-Arg-Glu-Lys-Arg-X ;
ii) un peptide de formule : 
(II) Y–Arg–Asp–Asn–Trp–Arg–Ser–Glu–Leu–Tyr–Lys–Tyr–
Lys–Val–Ile–Lys–Ile–Glu–Pro–Leu–Gly–Ile–Ala–Pro–
Thr–Lys–Ala–Lys–Arg–Arg–Val–Val–Gln–Arg–Glu–
Lys–Arg–X ;
iii) un peptide de formule : 
(III) Y-Thr-Arg-Asp-Gly-Gly-Asn-Ser-Asn-Glu-Ser-Glu-
Ile-Phe-Arg-Pro-Gly-Gly-Gly-Asp-Met-Arg-Asp-
Asn-Trp-Arg-Ser-Glu-Leu-Tyr-Lys-Tyr-Lys-Val-
Val-Lys-Ile-Glu-Pro-Leu-Gly-Val-Ala-Pro-Thr-Lys-
Ala-Lys-Arg-Arg-Val-Val-Gln-Arg-Glu-Lys-Arg-X ;
 et ses analogues par substitution, délétion et addition, où Y correspond à -H ou un acide aminé, et X est -OH, -NH₂ ou -NR₁R₂ (R étant un groupe alkyle), ledit procédé comprenant la préparation desdits peptides soit
a) en solution ou par une méthodologie de préparation de peptides en phase solide à l'aide de protocoles de couplage par étapes ou de fragments ;
b) par voie enzymatique ;
c) par synthèse, clonage et expression de régions de gènes codant lesdits peptides à l'aide d'une méthodologie de recombinaison d'ADN.

15. Procédé selon la revendication 14, partie (c), dans lequel les gènes sont sous-clonés et exprimés dans des cellules de E. coli, de levure ou de mammifères.

16. Procédé selon la revendication 14, dans lequel les peptides sont combinés à d'autres épitopes ou antigènes de HIV sous forme de produits de fusion.
